# EUROPEAN PATENT APPLICATION

(11) **EP 1 589 358 A1**
(43) Date of publication of application: **26.10.2005**
(21) Application number: 04704367.4
(22) Date of filing: 22.01.2004
(51) Int. Cl.: G02B 5/22, C09K 3/00, C07C 251/30

(54) **NEAR-INFRARED ABSORBING COMPOUND AND NEAR-INFRARED ABSORBING FILTER USING SAME**

(30) Priority: 27.01.2003 JP 2003017537
(71) Applicant: NIPPON KAYAKU KABUSHIKI KAISHA, Tokyo 102-8172 (JP)
(72) Inventor: KITAYAMA, Yasuyuki, Saitama-shi, Saitama 3300835 (JP); YAMAMURA, Shigeo, Ramiiyu Omiya Hairaizu, Saitama-shi, Saitama 3310812 (JP)
(74) Representative: Wablat, Wolfgang
(86) International application number: PCT/JP2004/000535
(87) International publication number: WO 2004/068199

(57) **Abstract**

A near-infrared absorbing filter which does not contain antimony, arsenic or the like and is excellent in heat resistance is disclosed. The near-infrared absorbing filter is **characterized by** containing a compound composed of a salt of cations obtained by oxidizing a substance represented by the formula (1) below and anions (X), which are alkylsulfonate ions having 1-8 carbon atoms that are necessary for neutralizing the cations and not substituted or may be substituted with a halogen atom, a lower alkyl group, a cyano group, or a hydroxy group. (1) (in the formula (1), rings A and B may have a substituent, and R₁-R₈ independently represent a substituted or non-substituted (C1 to C8) alkyl group, cycloalkyl group, alkenyl group or aryl group.)

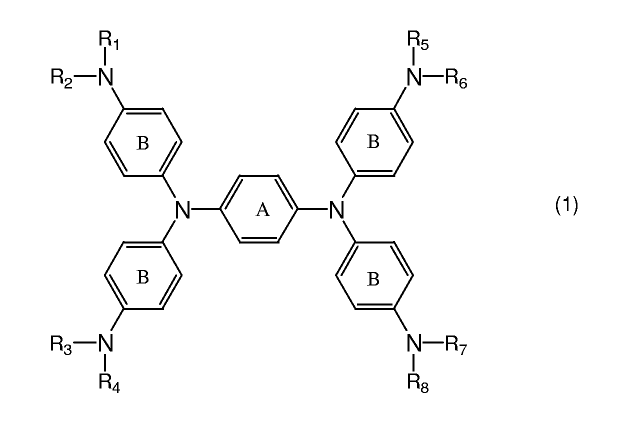

## Description

### Technical Field

The present invention relates to a near-infrared absorbing compound excellent in heat resistance without falling into a deleterious substance, a near-infrared absorbing filter, and a near-infrared absorbing composition, and particularly a near-infrared absorbing filter for a plasma display panel consisting of the above-described near-infrared absorbing filter.

### Background Art

Diimonium salt compounds and aminium salt compounds are heretofore broadly known as near-infrared absorbing agents (for example, see Japanese Patent Publication (KOKOKU) No. 7-51555 (page 2), Japanese Patent Application Laying Open (KOKAI) No. 10-316633 (page 5), and Japanese Patent Publication (KOKOKU) No. 43-25335 (pages 7-14)), and widely used, for example in a near-infrared absorbing filter, a heat insulating film, sunglasses, or the like. Among these compounds, those in which the counter ions are each a hexafluoroantimonate ion, a hexafluoroarsenic ion, or the like are excellent in heat resistance, and in particular such compounds of the hexafluoroantimonate ion have been mainly used. However, in industrial fields in which heavy metals are subjected to regulation, particularly in the electric material field, there has been a need for compounds that do not contain these metals because any compound just containing antimony is made to fall into a deleterious substance. Although there is a method using a perchlorateion, a hexafluorophospateion, a fluoroborateion, or the like as a means for satisfying the need, these counter ions are not sufficient, considering resistance to heat and to moist heat. Further, although compounds using naphthalenedisulfonate as a counter ion have been proposed (for example, see Japanese Patent Application Laying Open (KOKAI) No. 10-316633 (page 5)), they could not be practically used due to a reduced molar absorption coefficient and a greenish color.

Compounds using a trifluoromethanesulfonate ion as a counter ion are also known; however, their specific data have not been presented (for example, see Japanese Patent Publication (KOKOKU) No. 7-51555 (page 2)).

### Problems to be Solved by the Invention

Under these circumstances, the present invention is directed to providing a near-infrared absorbing compound which does not contain antimony and is excellent in stability, particularly in heat resistance compared to other antimony-free counter ions, and a near-infrared absorbing filter suitable for a plasma display panel, prepared using the near-infrared absorbing compound.

### Disclosure of the Invention

As the result of earnest efforts for solving the above described problems, the present inventors have found that a near-infrared absorbing compound having a structure of formula (1) below solves the problems to accomplish the present invention is accomplished.

Thus, the present invention relates to:
(1) a near-infrared absorbing filter characterized by comprising a compound consisting of a salt of a cation obtained by oxidation of a substance of formula (1) below and an anion: 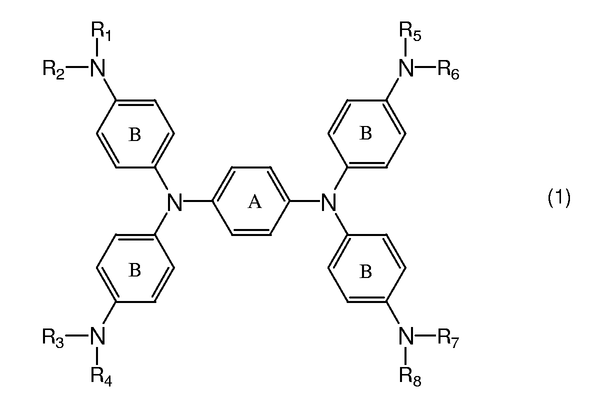 wherein rings A and B may have a substituent(s), and R₁ to R₈ independently represent a substituted or unsubstituted (C1 to C8) alkyl group, cycloalkyl group, alkenyl group or aryl group;
   said anion (X) being an alkylsulfonate ion having 1 to 8 carbon atoms, necessary for neutralization of the cation, which may be either unsubstituted or substituted with a halogen atom, a lower alkoxy group, cyano group or hydroxyl group;
(2) the near-infrared absorbing filter described in item (1), wherein the compound consisting of a salt of a cation obtained by oxidation of a substance of formula (1) and an anion has a structure of formula (4) below: 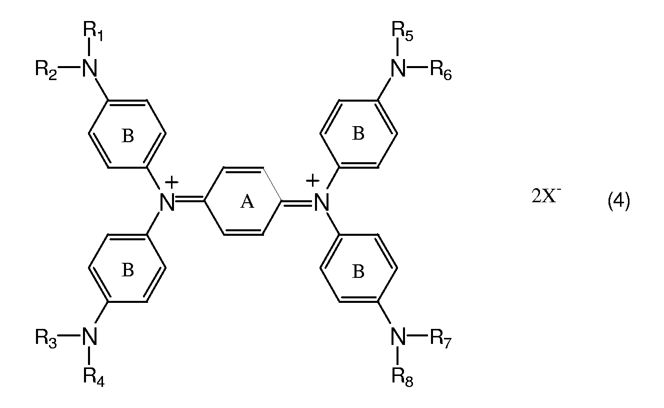
(3) the near-infrared absorbing filter described in item (1) or (2), wherein rings A and B are unsubstituted except in the 1- and 4-positions, or each have 1 to 4 halogen atoms, lower alkyl groups, lower alkoxy groupa, cyano groups or hydroxyl groups as substituents;
(4) the near-infrared absorbing filter described in any one of items (1) to (3), wherein X is an alkylsulfonic acid having 1 to 8 carbon atoms which is unsubstituted or substituted with a fluorine atom;
(5) the near-infrared absorbing filter described in any one of items (1) to (4), the filter is for use in a plasma display panel;
(6) a near-infrared absorbing composition characterized by comprising, in a resin, a compound consisting of a salt of a cation obtained by oxidation of a substance of formula (1) and an anion, said anion being an alkylsulfonate ion having 1 to 8 carbon atoms, necessary for neutralization of the cation, which may be either unsubstituted or substituted with a halogen atom, a lower alkoxy group, cyano group or hydroxyl group;
(7) a near-infrared absorbing compound consisting of a salt of a cation obtained by oxidation of a substance of formula (1) below and an anion: 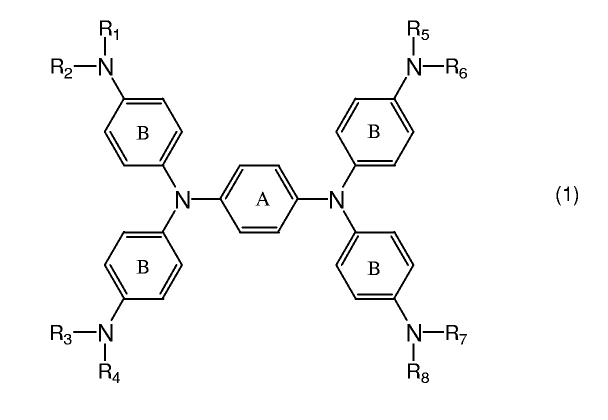 wherein rings A and B may have a substituent(s), and R₁ to R₈ independently represent a substituted or unsubstituted (C1 to C8) alkyl group, cycloalkyl group, alkenyl group or aryl group;
   said anion being an alkylsulfonic acid, necessary for neutralization of the cation, represented by formula (2) below: 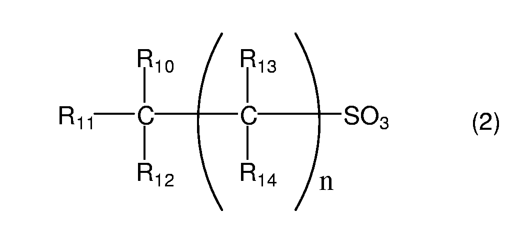 wherein R₁₀ to R₁₄ independently represent a hydrogen or halogen atom, a lower alkyl group, lower alkoxy group, cyano group or hydroxyl group, and n represents an integer of 1 to 7; and
(8) a near-infrared absorbing compound represented by formula (6) below: 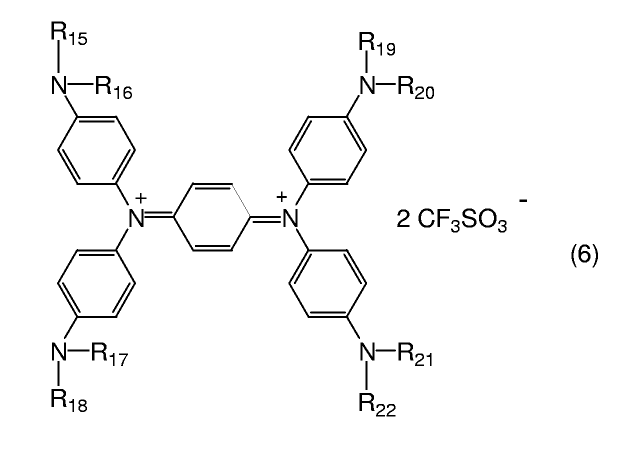 wherein R₁₅ to R₂₂ independently represent a straight-chain or branched butyl or pentyl group.

### Best Mode for Carrying Out the Invention

The near-infrared absorbing filter of the present inventtion is obtained by applying the compound having a structure represented by formula (1) above. The example of such compound is the compound represented by chemical formula (3) below: 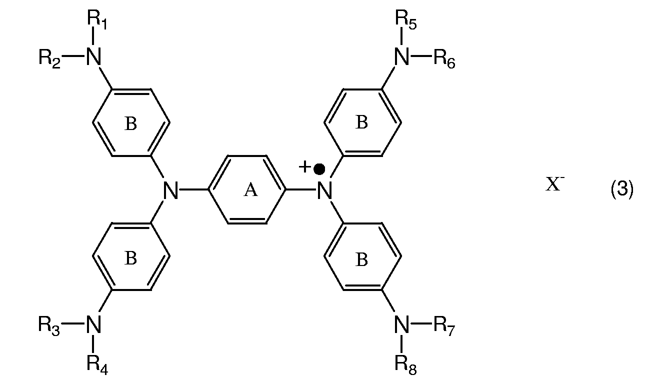 wherein rings A and B, R₁ to R₈, and X are as described above or chemical formula (4) below: 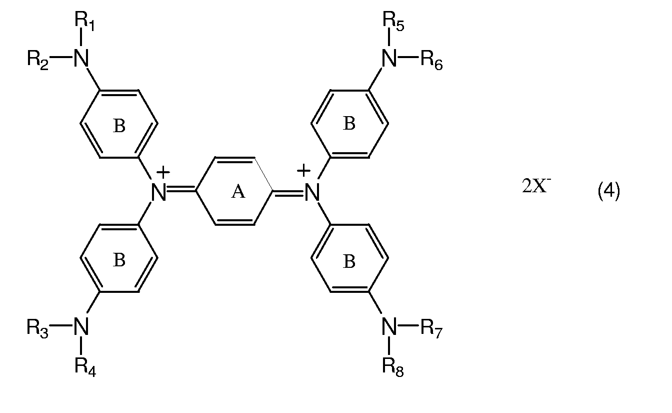 wherein rings A and B, R₁ to R₈, and X are as described above.

In general formula (3) and/or (4), each of rings A and B may have, or may not have 1 to 4 substituents except in the 1- and 4-positions. Substituents which may be bound include halogen atoms, and hydroxyl, lower alkoxy, cyano, and lower alkyl groups. The halogen atommaybe, for example, a fluorine, chlorine, bromine, or iodine atom, or the like. The alkoxy group may be, for example, a C1 to C5 alkoxy group such as methoxy, ethoxy, or the like., and the lower alkyl group may be, for example, a C1 to C5 alkyl group such as methyl, ethyl, or the like. Preferably, rings A and B are unsubstituted or substituted with a halogen atom (particularly, chlorine or bromine atom) or a methyl or cyano group.

In this respect, it is preferable for synthesis that when ring B have a substituent, all of the four B rings have identical substituents, preferably in the meta-position with respect to the nitrogen atom binding to the A ring. In addition, it is preferable for synthesis that rings A and B are unsubstituted except in the 1- and 4-positions.

In R₁ to R₈, the alkyl group may be, for example, methyl, ethyl, propyl, butyl, pentyl, or the like. The alkyl moiety may be straight chain or branched, and may be also substituted. Substituents which may be bound include halogen atoms (e.g., F, Cl, and Br) and hydroxy, alkoxy (e.g., methoxy, ethoxy, isobutoxy, and the like), alkoxyalkoxy (e.g., methoxyethoxy and the like), aryl (e.g., phenyl, naphtyl, and the like), arylxy (e.g., phenoxy and the like), acyloxy (e.g., acetyloxy, butylyloxy, hexylyloxy, benzoyloxy, and the like), alkyl-substituted amino (e.g., methylamino, dimethylamino, and the like), cyano, nitro, carboxyl, and sulfo groups.

The cycloalkyl group may be, for example, cyclopentyl, cyclohexyl, or the like. The alkenyl group may be, for example, allyl, 1-butenyl, 1-pentenyl, or the like. The aryl group may be, for example, phenyl, naphtyl, or the like. The aryl group may be substituted. The substituent may be, for example, an alkyl group having 1 to 8 carbon atoms (e.g., methyl, ethyl, butyl, or the like), an alkoxy group having 1 to 6 carbon atoms (e.g., methoxy, ethoxy, or the like), an aryloxy group (e.g., phenoxy, p-chlorophenoxy, or the like), a halogen atom (e.g., F, Cl or Br), an alkoxycarbonyl group (e.g., methoxycarbonyl, ethoxycarbonyl, or the like), an amino group, an alkylsubstituted amino group (e.g., methylamino or the like), an amide group (e.g., acetamide or the like), a sulfonamide group (e.g., methanesulfonamide or the like), a cyano group, a nitro group, a carboxyl group, a sulfo group, or the like. Preferably, the aryl group has 6 to 12 carbons.

Preferred R₁ to R₈ are each an unsubstituted alkyl group, a cyano-substituted alkyl group, an alkoxy-substituted alkyl group, or an aryl group. Particularly, they are each a (C1 to C8) alkyl group such as methyl, ethyl, n-propyl, isopropyl, n-butyl, sec-butyl, isobutyl, pentyl, isopentyl, hexyl, heptyl, or the like, a cyano-substituted (C1 to C6) alkyl group such as cyanomethyl, 2-cyanoethyl, 3-cyanopropyl, 2-cyanopropyl, 4-cyanobutyl, 3-cyanobutyl, 2-cyanobutyl, 5-cyanopentyl, 4-cyanopentyl, 3-cyanopentyl, 2-cyanopentyl, or the like, or an alkoxy-substituted (C1 to C6) alkyl group such as methoxyethyl, ethoxyethyl, 3-methoxypropyl, 3-ethoxypropyl, 4-methoxybutyl, 4-ethoxybutyl, 5-ethoxypentyl, 5-methoxypentyl, or the like, and n-butyl, isobutyl, n-pentyl, or isopentyl is particularly preferable.

X represents an alkylsulfonic acid having 1 to 8 carbon atoms, necessary for neutralization of the cation (electrical charge) obtained by oxidation of a compound of formula (1), which may be unsubstituted or substituted with a halogen atom, a lower alkoxy group, a cyano group, or a hydroxy group, and may be a straight or branched chain. For neutralization of the electrical charge, one molecule of a compound of formula (3) or two molecules of a compound of formula (4) is required. Specific examples of the alkylsulfonic acidhaving 1 to 8 carbon atoms include, for example, methanesulfonic acid, ethanesulfonic acid, propanesulfonic acid, butanesulfonic acid, pentanesulfonic acid, hexanesulfonic acid, heptanesulfonic acid, nonanesulfonic acid, and the like. They may be substituted with the above described halogen atom, cyano group, or hydroxyl group. The compound substituted with a halogen atom may be, for example, trifluoromethanesulfonic acid, trichloromethanesulfonic acid, pentafluoroethanesulfonic acid, 2-bromoethanesulfonic acid, 2-chloroethanesulfonic acid, heptafluoropropanesulfonic acid, 3-bromopropanesulfonic acid, 3-chloropropanesulfonic acid, nonafluorobutanesulfonic acid, heptadecafluorooctanesulfonic acid, or the like; the compound substituted with a cyano group may be, for example, cyanomethanesulfonic acid, 2-cyanoethanesulfonic acid, 4-cyanobutanesulfonic acid, or the like; and the compound substituted with a hydroxyl group may be, for example, hydroxymethanesulfonic acid, 2-hydroxyethanesulfonic acid, 4-hydroxybutanesulfonic acid, or the like. Preferably, X is an alkylsulfonic acid unsubstituted, or substituted with the halogen atom, and the halogen atom is preferably fluorine.

Of these, methanesulfonic acid, ethanesulfonic acid, propanesulfonic acid,butanesulfonic acid,trifluoromethanesulfonic acid, pentafluoroethanesulfonic acid, heptafluoropropanesulfonic acid, and nonafluorobutanesufonate are particularly preferable.

Now, with regard to the near-infrared absorbing compound, specific examples of formula (3) are shown in Tables 1 to 3, and those of formula (4) in Tables 4 to 6. With R₁ to R₈ in Tables 1 to 6, "i-" stands for a branched state as "iso-", "Ph" for a phenyl group, and "cy" for "cyclic". With A and B, unsubstitution except in the 1- and 4-positions is indicated with "4H", and the substitution position represents the site of substitution relative to the nitrogen atom binding to the A ring. Also, R₁ to R₈ are abbreviated to "4 (n-C₄H₉, n-C₄H₉)" when all of them are butyl groups, and to "3 (n-C₄H₉, n-C₄H₉) (n-C₄H₉, i-C₅H₁₁)" when one is a iso-pentyl group and the others aren-butyl groups, that is, when one of the four combinations of substituents contains iso-pentyl and all of the remaining three combinations consist of an n-butyl group.

**Table 1**

| No. | (R1,R2) (R3,R4) (R5,R6) (R7,R8) | A | B | X |
|---|---|---|---|---|
| 1 | 4 (n-C4H9, n-C4H9) | 4H | 4H | CF3SO3 |
| 2 | 4 (i-C4H9, i-C4H9) | 4H | 4H | CF3SO3 |
| 3 | 4 (i-C5H11, i-C5H11) | 4H | 4H | CF3SO3 |
| 4 | 4(C2H4OCH3, C2H4OCH3) | 4H | 4H | CF3SO3 |
| 5 | 4 (CH2CH2CH2CN, CH2CH2CH2CN) | 4H | 4H | CF3SO3 |
| 6 | 4(CH2CH=CH2, CH2CH=CH2) | 4H | 4H | BrCH2SO3 |
| 7 | 4(CH2CH2CH2CH2CN, CH2CH2CH2CH2CN) | 4H | 4H | CF3SO3 |
| 8 | 4(n-C3H7,n-C3H7) | 4H | 4H | CF3SO3 |
| 9 | 4(n-C2H5,n-C2H5) | 4H | 4H | NCCH2SO3 |
| 10 | 4(n-C3H6COOH, n-C3H6COOH) | 4H | 4H | ClCH2SO3 |
| 11 | 4 (cy-C6H11, cy-C6H11) | 4H | 4H | CF3SO3 |
| 12 | 4(Ph,Ph) | 4H | 4H | HOCH2SO3 |

**Table 2**

| No. | (R1,R2) (R3,R4) (R5,R6) (R7,R8) | A | B | X |
|---|---|---|---|---|
| 13 | 4(n-C4H9, n-C4H9) | 4H | 4H | CF3 (CF2) 3SO3 |
| 14 | 4(i-C4H9, i-C4H9) | 4H | 4H | CF3 (CF2) 3SO3 |
| 15 | 4(i-C5H11, i-C5H11) | 4H | 4H | CF3 (CF2) 3SO3 |
| 16 | 4(C2H4OCH3, C2H4OCH3) | 4H | 4H | CF3 (CF2) 3SO3 |
| 17 | 4(CH2CH2CH2CN, CH2CH2CH2CN) | 4H | 4H | CF3 (CF2) 3SO3 |
| 18 | 4(CH2CH=CH2, CH2CH=CH2) | 4H | 4H | C2H5SO3 |
| 19 | 4((CH2CH2CH2CN, CH2CH2CH2CN) | 4H | 4H | CF3 (CF2) 7SO3 |
| 20 | 4(n-C4H9, n-C4H9) | 4H | 4H | CF3 (CF2) 7SO3 |
| 21 | 4(n-C2H5, n-C2H5) | 4H | 4H | CF3 (CF2) 2SO3 |
| 22 | 4(n-C3H6COOH, n-C3H6COOH) | 4H | 4H | n-C3H7SO3 |
| 23 | 4(cy-C6H11, cy-C6H11) | 4H | 4H | n-C4H9SO3 |
| 24 | 4(Ph-CH3,Ph-CH3) | 4H | 4H | CF3 (CF2) 3SO3 |

**Table 3**

| No | (R1,R2) (R3,R4) (R5,R6) (R7,R8) | A | B | X |
|---|---|---|---|---|
| 25 | 3(CH2CH2CH2CN, CH2CH2CH2CN) (CH2 CH2CH2CN,n-C4H9) | 4H | 4H | CF3(CF2)3SO3 |
| 26 | 3(n-C4H9, n-C4H9) (n-C4H9, C2H40C H3) | 4H | 4H | CF3(CF2)3SO3 |
| 27 | 3(C2H4OCH3, C2H4OCH3)(C2H4OCH3, CH2CH2CH2CN) | 4H | 4H | CF3(CF2)3SO3 |
| 28 | 3(n-C4H9, n-C4H9) (n-C4H9,i-C5H1 1) | 4H | 4H | CF3(CF2)3SO3 |
| 29 | 4 (n-C4H9, n-C4H9) | o-Cl | 4H | CF3(CF2)3SO3 |
| 30 | 4 (i-C4H9, i-C4H9) | 4H | o-CH3 | CH3SO3 |
| 31 | 4 (n-C4H9, n-C4H9) | 4H | o-CN | CF3SO3 |
| 32 | 4(CH2CH2CH2CN, CH2CH2CH2CN) | o-Cl | 4H | CF3SO3 |
| 33 | 3(CH2CH2CH2CN,CH2CH2CH2CN)(CH2 CH2CH2CN,n-C4H9) | o-Cl | 4H | CF3SO3 |
| 34 | 3(n-C4H9, n-C4H9) (n-C4H9, C2H40CH3) | 4H | o-CH3 | CH3SO3 |
| 35 | 3(C2H4OCH3,C2H4OCH3)(C2H4OCH3, CH2CH2CH2CN) | 4H | o-CN | BrCH2SO3 |
| 36 | 3(n-C4H9, n-C4H9) (n-C4H9, C2H4OCH3) | o-Cl | 4H | CF3(CF2)3SO3 |

**Table 4**

| No. | (R1,R2) (R3,R4) (R5,R6) (R7,R8) | A | B | X |
|---|---|---|---|---|
| 37 | 4(n-C4H9, n-C4H9) | 4H | 4H | CF3SO3 |
| 38 | 4(i-C4H9, i-C4H9) | 4H | 4H | CF3SO3 |
| 39 | 4(i-C5H11, i-C5H11) | 4H | 4H | CF3SO3 |
| 40 | 4(C2H40CH3, C2H4OCH3) | 4H | 4H | CF3SO3 |
| 41 | 4(CH2CH2CH2CN, CH2CH2CH2CN) | 4H | 4H | CF3SO3 |
| 42 | 4(CH2CH=CH2, CH2CH=CH2) | 4H | 4H | BrCH2SO3 |
| 43 | 4(CH2CH2CH2CH2CN, CH2CH2CH2CH2CN) | 4H | 4H | CF3SO3 |
| 44 | 4(n-C3H7,n-C3H7) | 4H | 4H | CF3SO3 |
| 45 | 4(n-C2H5,n-C2H5) | 4H | 4H | NCCH2SO3 |
| 46 | 4(n-C3H6COOH, n-C3H6COOH) | 4H | 4H | ClCH2SO3 |
| 47 | 4(cy-C6H11, cy-C6H11) | 4H | 4H | CF3SO3 |
| 48 | 4(Ph,Ph) | 4H | 4H | HOCH2SO3 |

**Table 5**

| No. | (R1,R2) (R3,R4) (R5,R6) (R7,R8) | A | B | X |
|---|---|---|---|---|
| 49 | 4(n-C4H9, n-C4H9) | 4H | 4H | CF3 (CF2) 3SO3 |
| 50 | 4(i-C4H9, i-C4H9) | 4H | 4H | CF3 (CF2) 3SO3 |
| 51 | 4(i-C5H11, i-C5H11) | 4H | 4H | CF3 (CF2) 3SO3 |
| 52 | 4(C2H4OCH3, C2H4OCH3) | 4H | 4H | CF3 (CF2) 3SO3 |
| 53 | 4(CH2CH2CH2CN, CH2CH2CH2CN) | 4H | 4H | CF3 (CF2) 3SO3 |
| 54 | 4(CH2CH=CH2, CH2CH=CH2) | 4H | 4H | C2H5SO3 |
| 55 | 4((CH2CH2CH2CN, (CH2CH2CH2CN) | 4H | 4H | CF3 (CF2) 7SO3 |
| 56 | 4(n-C4H9, n-C4H9) | 4H | 4H | CF3 (CF2) 7SO3 |
| 57 | 4(n-C2H5, n-C2H5) | 4H | 4H | CF3 (CF2) 2SO3 |
| 58 | 4(n-C3H6COOH, n-C3H6COOH) | 4H | 4H | n-C3H7SO3 |
| 59 | 4(cy-C6H11, cy-C6H11) | 4H | 4H | n-C4H9SO3 |
| 60 | 4(Ph-CH3,Ph-CH3) | 4H | 4H | CF3 (CF2) 3SO3 |

**Table 6**

| No. | (R1,R2) (R3,R4) (R5,R6) (R7,R8) | A | B | X |
|---|---|---|---|---|
| 61 | 3(CH2CH2CH2CN, CH2CH2CH2CN) (CH2CH2CH2CN,n-C4H9) | 4H | 4H | CF3(CF2)3SO3 |
| 62 | 3(n-C4H9, n-C4H9) (n-C4H9, C2H4OCH3) | 4H | 4H | CF3(CF2)3SO3 |
| 63 | 3(C2H4OCH3, C2H4OCH3) (C2H4O CH3,CH2CH2CH2CN) | 4H | 4H | CF3(CF2)3SO3 |
| 64 | 3(n-C4H9, n-C4H9) (n-C4H9, C2 H40CH3) | 4H | 4H | CF3(CF2)3SO3 |
| 65 | 4(n-C4H9, n-C4H9) | o-C 1 | 4H | CF3(CF2)3SO3 |
| 66 | 4(i-C4H9, i-C4H9) | 4H | o-CH3 | CH3SO3 |
| 67 | 4(n-C4H9, n-C4H9) | 4H | o-C N | CF3SO3 |
| 68 | 4(CH2CH2CH2CN, CH2CH2CH2CN) | o-C 1 | 4H | CF3SO3 |
| 69 | 3(CH2CH2CH2CN, CH2CH2CH2CN) (CH2CH2CH2CN, n-C4H9) | o-C 1 | 4H | CF3SO3 |
| 70 | 3(n-C4H9, n-C4H9) (n-C4H9, C2H4OCH3) | 4H | o-CH3 | CH2SO3 |
| 71 | 3(C2H4OCH3,C2H4OCH3) (C2H4O CH3, CH2CH2CH2CN) | 4H | o-C N | BrCH2SO3 |
| 72 | 3 (n-C4H9, n-C4H9) (n-C4H9, C2 H40CH3) | o-Cl | 4H | CF3(CF2)3SO3 |
| 73 | 3(i-C4H9, i-C4H9) (i-C4H9, n-C4H9) | 4H | 4H | CF3SO3 |

The compounds represented by general formulas (3) and/or (4), finding use in the near-infrared absorbing filter of the invention may be produced by a method complying with, for example, the method described in Japanese Patent Publication (KOKOKU) No. 43-25335 (pages 7-14)). Specifically, the product obtained by subjecting p-phenylenediamine and 1-chloro-4-nitrobenzene to Ullmann reaction may be reduced, followed by reacting the resultant amino compound of formula (5) below: 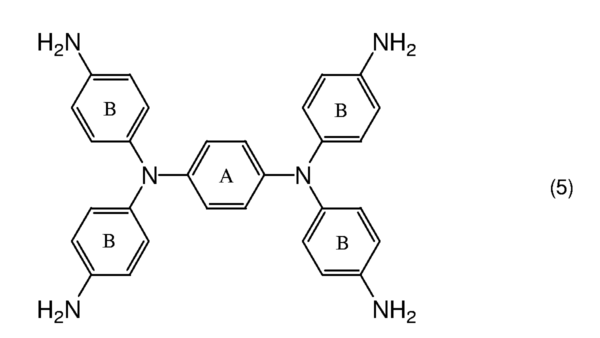 wherein rings A and B are as defined above with a halogenated compound(s) corresponding to desired R₁ to R₈ (for example, BrC₄H₉ if R is n-C₄H₉) in an organic solvent, preferably a water-soluble polar solvent such as DMF, DMI, or NMP, at 30 to 160°C, preferably 50 to 140°C to provide a compound inwhich all of the substituents (R₁ to R₈) are identical (hereinafter referred to as an entirely substituted compound). In order to synthesize a compound other than that in which R₁ to R₈ are all identical substituents (for example, the compound of No. 28), the reaction with given moles (7 moles per mole of the amine compound of formula (5) above) of a reagent (BrC₄H₉) is first performed to introduce n-butyl groups into seven groups of R₁ to R₈, followed by the reaction with necessary moles of a corresponding agent (BrC₅H₁₁) for introducing the remaining substituent (an i-pentyl group) (1 mole per mole of the amine compound of formula (5) above). Any compound other than the entirely substituted compound may be produced using a similar process to that for preparing the compound of No. 28 exemplified above.

Subsequently, an oxidizing agent corresponding to X of formula (3) or (4) (for example, a silver salt) is added to the compound synthesized above for oxidation reaction in a water-soluble polar solvent such as DMF, DMI, or NMP at 0 to 100°C, preferably 5 to 70°C. Generally, use of two equivalents of the oxidizing agent provides the compound of general formula (4) ; one equivalent gives the compound of general formula (3) .

The compound of general formula (3) or (4) may be also synthesized using a method involving oxidizing the compound synthesized above with an oxidizing agent such as silver nitrate, silver perchlorate, or cupric chloride, followed by adding an acid or salt of a desired anion to the reaction liquid for salt exchange.

The near-infrared absorbing filter of the invention may be provided with a layer containing the above described near-infrared absorbing compound on a substrate, or the substrate itself may be a layer consisting of a resin composition (or the cured matter thereof) containing the near-infrared absorbing compound. The substrate is particularly not restricted as far as it can be generally used for a near-infrared absorbing filter; however, a substrate made of a resin is typically used. The thickness of the layer containing the near-infrared absorbing compound is generally on the order of 0.1 **µ**m to 10 mm, although it is properly determined according to a specific purpose such as for a near-infrared ray cutting rate. The content of the near-infrared absorbing compound is also determined appropriately depending on a desired near-infrared ray cutting rate.

The resin providing the substrate preferably has maximal transparency when molded into a resin plate or film, and specific examples of the resin include vinyl compounds such as polyethylene, polystyrene, polyacrylic acid, polyacrylate, polyvinyl acetate, polyacrylonitrile, polyvinyl chloride, and polyvinyl fluoride, or addition polymers thereof, polymethacrylic acid, polymethacrylate, polyvinylidene chloride, polyvinylidene fluoride, polyvinylidene cyanide, copolymers of vinyl compounds or fluorine-containing compounds such as vinylidene fluoride/trifluoroethylene copolymer, vinylidene fluoride/tetrafluoroethylene copolymer, and vinylidene cyanide/vinyl acetate copolymer, fluorine-containing resins such as polytrifluoroethylene, polytetrafluoroethylene, and polyhexafluoropropylene, polyamides such as nylon 6 and nylon 66, polyimide, polyurethane, polypeptide, polyesters such as polyethylene terephthalate, polycarbonate, polyethers such as polyoxymethylene, epoxy resin, polyvinyl alcohol, polyvinyl butyral, and the like.

Methods for preparing the near-infrared absorbing filter of the invention are particularly not limited, and may be, for example, the following methods which are well known per se: (1) kneading a resin with the near-infrared absorbing compound of the invention, followed by heating and forming to prepare a resin plate or film; (2) subjecting the above compound and a resin monomer(s) or a prepolymer thereof to cast polymerization in the presence of a polymerization catalyst to prepare a resin plate or film; (3) preparing a paint containing the above compound, followed by coating a transparent resin plate, a transparent film, or a transparent glass plate with the paint; and (4) including the compound in an adhesive, followed by preparing a laminated resin plate, a laminated resin film, or a laminated glass plate.

The preparing method of (1) may be typically, for example, a method wherein the near-infrared absorbing compound of the invention is added to a powder or pellet of substrate resin before heating and dissolving at 150 to 350°C, followed by molding to prepare a resin plate, or forming into a film (or resin plate) by extruder, although processing temperature, film-forming (resin plate-forming) conditions, or the like vary slightly depending on what resins are used. The amount of the near-infrared absorbing compound added is generally 0.01 to 30% by weight, preferably 0.03 to 15% by weight, based on the weight of a binder resin although it varies depending on the thickness, absorption intensity, visual light transmittance, or the like of the resin plate or film to be prepared.

In the method of (2), wherein the above compound and a resin monomer (s) or a prepolymer thereof are subjected to cast polymerization in the presence of a polymerization catalyst for the preparation, molding is carried out by injecting their mixture into a mold to allow to react for curing, or by casting the mixture in a die, followed by setting until it becomes a hard product. Most resins can be molded during these processes, and specific examples of such resins include acrylic resin, diethylene glycol bis (allyl carbonate) resin, epoxy resin, phenol-formaldehyde resin, polystyrene resin, silicone resin, and the like. Above all, the casting method using the bulk polymerization of methyl methacrylate which provides an acrylic sheet excellent in hardness, heat resistance and chemical resistance is preferable.

As a polymerization catalyst, a known radical thermal polymerization initiator may be used, and examples of the initiator include peroxides such as benzoyl peroxide, p-chlorobenzoyl peroxide, and diisopropylperoxy carbonate, and azo compounds such as azobisisobutylonitrile. The usage quantity thereof is generally 0.01 to 5% by weight based on the total quantity of the mixture. The heating temperature for thermal polymerization is generally 40 to 200°C, and the polymerization time is generally on the order of 30 minutes to 8 hours. In addition to the thermal polymerization, a method involving addition of a photopolymerization initiator or a sensitizing agent for photopolymerization may be also used.

As the method of (3), there are, for example, a method involving dissolving the near-infrared absorbing compound of the invention in a binder resin and an organic solvent so as to form into a paint, and a method involving finely pulverizing the above compound for dispersion to form a water-based paint. The former method may use, as a binder, for example, aliphatic ester resin, acrylic resin, melamine resin, urethane resin, aromatic ester resin, polycarbonate resin, polyvinyl resin, aliphatic polyolefin resin, aromatic polyolefin resin, polyvinyl alcohol resin, polyvinyl modified resin, or the like, or a copolymer resin thereof.

As a solvent, a halogen-, alcohol-, ketone-, ester-, aliphatic hydrocarbon-, aromatic hydrocarbon-, or ether-based solvent, or a mixture thereof may be used. The concentration of the near-infrared absorbing compound of the invention is typically 0.1 to 30% by weight to the binder resin although it varies depending on the thickness, absorption intensity, or visual light transmittance of a coating to be prepared.

The paint thus prepared may be employed to coat a transparent resin film, a transparent resin plate, a transparent glass, or the like using a spin coater, a bar coater, a roll coater, a spray, or the like to provide a near-infrared absorbing filter.

In the method of (4), the adhesive may use a known transparent adhesive, i.e. a silicone-, urethane-, or acrylic-based adhesive, or the like for resin, a polyvinyl butyral adhesive for laminated glass, or an ethylene-vinyl acetate-based adhesive, or the like for laminated glass. An adhesive having 0.1 to 30% by weight of the near-infrared absorbing compound of the invention added is used to bond transparent resin plates together, a resin plate and a resin film in combination, a resin plate and a glass in combination, resin films together, a resin film and a glass in combination, or glasses together to prepare a filter.

In this respect, conventional additives used for resin molding such as an ultraviolet absorber and a plasticizer may be added in kneading and mixing in the respective methods.

Near-infrared absorbing compositions obtained by adding compounds represented by formulas (3) and/or (4) into resins in the respective methods of (1) to (4) are also intended to be embraced in the present invention.

In order to change the color tone of the filter, a coloring matter (a dye for color matching) with absorption in a visible ray region may be added as far as advantages of the invention is not impaired. Alternatively, a filter containing only a dye for color matching may be prepared, followed by laminating, on this filter, the near-infrared absorbing filter.

When used in the front plate of a plasma display, such a near-infrared absorbing filter preferably has a visible light transmittance as high as possible, which needs to be at least 40%, preferably 50% or higher. The cut region of a near-infrared ray is preferably 800 to 900 nm, more preferably 800 to 1, 000 nm, and the average transmittance to the near-infrared ray in the region is preferably 50% or lower, more preferably 30% or lower, further preferably 20% or lower, particularly 10% or lower.

According to the invention, the compound of formula (4), which tends to have a high transmittance to visible light, is preferably used although the compound of formula (3) or a mixture of the compounds of formulas (3) and (4) may be used. In addition, combinations of these compounds and other near-infrared absorbing compounds may be used for the preparation. Other near-infrared absorbing compounds usable in combination include, for example, phthalocyanine-based dyes, cyanine-based dyes, dithiol nickel complexes, and the like. Usable near-infrared absorbing compounds of inorganic metals include, for example, metal copper, copper compounds such as copper sulfide and copper oxide, metal mixtures consisting mainly of zinc oxide, tungsten compounds, ITO, ATO, and the like.

The near-infrared absorbing filter of the invention can be used not only in applications such as the front plate of a display, but also in a filter or film requiring the cutting of an infrared ray, for example, a heat-insulating film, optical goods, sunglasses, or the like.

The near-infrared absorbing filter of the invention is an excellent near-infrared absorbing filter which has an extremely high transmittance in the visible light region, contains no antimony, and shows a wide absorption in the near-infrared region. The filter is also excellent in stability compared to a conventional near-infrared absorbing filter comprising a perchlorate ion, a hexafluorophosphate ion, or a fluoroborate ion without containing antimony. Particularly, the near-infrared absorbing filter of the invention is highly excellent in heat resistance, and produces little coloration in the visible region because it less easily causes reactions such as degradation due to heat. Due to these features, the filter can be suitably used in near-infrared absorbing filters or near-infrared absorbing films such as, for example, a heat-insulating film and sunglasses, and particularly fits for a near-infrared absorbing filter for a plasma display.

### Examples

The present invention is further concretely described below with reference to Examples. However, the invention is not intended to be limited by these Examples. In Examples, "part" stands for "part by weight" unless otherwise specified.

### Example 1 (Synthesis Example 1)

### (Synthesis of the Compound of No. 37 in Table 4)

Into 10 parts of DMF was added 1.8 parts of N,N,N',N'-tetrakis{p-di(n-butyl)aminophenyl}-p-phenylened iamine which was then heated to 60°C for dissolution, followed by adding 1.08 parts of silver trifluoromethanesulfonate dissolved in 10 parts of DMF before reaction for 30 minutes. After cooling, the deposited silver was filtered off. To the reaction liquid (filtrate) was slowly added dropwise 20 parts of water, followed by stirring for 15 minutes. The generated black crystal was filtrated and washed with 50 parts of water, followed by drying the resultant cake to provide 2.3 parts of the compound of No. 37.
λmax: 1,100 nm (dichloromethane)

### Synthesis Example 2

### (Synthesis of the Compound of No. 39 in Table 4)

The same reaction was carried out as that in Example 1 except for the use of
N,N,N',N'-tetrakis {p-di (i-amyl) aminophenyl}-p-phenylenedi amine in place of
N,N,N',N'-tetrakis{p-di(n-butyl)aminophenyl}-p-phenylened iamine to provide the compound of No. 39.
λmax: 1,104 nm (dichloromethane)

### Synthesis Example 3

### (Synthesis of the Compound of No. 38 in Table 4)

The same reaction was carried out as that in Example 1 except for the use of
N,N,N',N'-tetrakis{p-di (i-butyl)aminophenyl}-p-phenylened iamine in place of
N,N,N',N'-tetrakis {p-di (n-butyl) aminophenyl} -p-phenylened iamine to provide the compound of No. 38.
λmax: 1,106 nm (dichloromethane)

### Synthesis Example 4

### (Synthesis of the Compound of No. 41 in Table 4)

The same reaction was carried out as that in Example 1 except for the use of
N,N,N',N'-tetrakis{p-di (cyanopropyl) aminophenyl}-p-phenylenediamine in place of
N,N,N',N'-tetrakis {p-di (n-butyl) aminophenyl}-p-phenylened iamine to provide the compound of No. 41.
λmax: 1,068 nm (dichloromethane)

### Synthesis Example 5

### (Synthesis of the Compound of No. 5 in Table 1)

The same reaction was carried out as that in Example 1 except for the use of
N,N,N',N'-tetrakis {p-di (cyanopropyl) aminophenyl}-p-phenyl enediamine in place of
N,N,N',N'-tetrakis {p-di (n-butyl) aminophenyl}-p-phenylened iamine and the changing of the usage quantity of silver trifluoromethanesulfonate into one equivalent to provide the compound of No. 5.
λmax: 880 nm (acetone)

### Example 2 (Synthesis Example 6)

### (Synthesis of the Compound of No. 49 in Table 5)

Into 17 parts of DMF was added 3 parts of N,N,N',N'-tetrakis{p-di(n-butyl)aminophenyl}-p-phenylened iamine and 2.3 parts of potassium nonafluorobutanesulfonate which were then heated to 60°C for dissolution, followed by adding 1.2 parts of silver nitrate dissolved in 17 parts of DMF before reaction for one hour. After cooling, the deposited silver was filtered off. To the reaction liquid (filtrate) was slowly added dropwise 35 parts of water, followed by stirring for 15 minutes. The generated black crystal was filtrated and washed with 50 parts of water, followed by drying the resultant cake to provide 4.6 parts of the compound of No. 49.
λmax: 1,100 nm (dichloromethane)

### Example 3 (Synthesis Example 7)

### (Synthesis of the Compound of No. 17 in Table 2)

Into 17 parts of DMF was added 3 parts of N,N,N',N'-tetrakis {p-di (cyanopropyl) aminophenyl}-p-phenyl enediamine and 1 part of potassium nonafluorobutanesulfonate which were then heated to 60°C for dissolution, followed by adding 0.5 part of silver nitrate dissolved in 17 parts of DMF before reaction for one hour. After cooling, the deposited silver was filtered off. To the reaction liquid (filtrate) was slowly added dropwise 35 parts of water, followed by stirring for 15 minutes. The generated black crystal was filtrated and washed with 50 parts of water, followed by drying the resultant cake to provide 3.6 parts of the compound of No. 17.
λmax: 882 nm (acetone)

### Example 4 (Synthesis Example 8)

### (Synthesis of the Compound of No. 56 in Table 5)

The same reaction was carried out as that in Example 2 except for the use of tetraethylammonium heptadecafluorooctanesulfonate in place of potassium nonafluorobutanesulfonate to provide the compound of No. 56.
λmax: 1,098 nm (dichloromethane)

### Example 5 (Synthesis Example 9)

### (Synthesis of the Compound of No. 19 in Table 2)

The same reaction was carried out as that in Example 3 except for the use of tetraethylammonium heptadecafluorooctanesulfonate in place of potassium nonafluorobutanesulfonate to provide the compound of No. 19.
λmax: 884 nm (acetone)

### Synthesis Example 10

### (Synthesis of the Compound of No. 73 in Table 6)

Into 35 parts of DMF was added 5.3 parts of N,N,N',N'-tetrakis(aminophenyl)-p-phenylenediamine, 20 parts of potassium carbonate, 10 parts of potassium iodide, 5 parts of n-butylbromide, and 35 parts of isobutylbromide for reaction at 90°C for three hours, followed by reaction at 130°C for one hour. After cooling, liquid filtration was carried out, and 40 parts of methanol was added to the reaction liquid, followed by stirring at 5°C or lower for one hour. The generated crystal was filtrated, washed with methanol, and then dried to provide 7.1 parts of an intermediate as a light brown crystal.

The same reaction was carried out as that in Example 1 except for the use of the intermediate obtained by the above substitution reaction in place of
N,N,N',N'-tetrakis{p-di(n-butyl)aminophenyl}-p-phenylened iamine to provide the compound of No. 73.
λmax: 1,104 nm (dichloromethane)

As are the cases with Synthesis Examples 1 to 8, the other listed compounds may be synthesized by oxidizing corresponding phenylenediamine derivatives using the above various oxidizing agents including silver salts corresponding to X, followed by reaction with corresponding anions.

### Examples 6 and 7

For each of the compounds obtained in Examples above, a molar absorbance coefficient (ε) was determined in dichloromethane. The determination of a molar absorbance coefficient was carried out on the compound of No. 37 in Example 6, and on the compound of No. 49 in Example 7. The results obtained are shown in Table 7.

### Comparative Examples 1 and 2

Molar absorbance coefficients (ε) were determined in dichloromethane in the same way as in the preceding Examples except for the use of
N,N,N',N'-tetrakis{p-di(n-butyl)aminophenyl}phenylenediim monium 1, 5-naphthalenedisulfonate (the compound described in Japanese Patent Application Laying Open (KOKAI) No. 10-316633 (page 5) or Example 1) (Comparative Example 1) and a 1-hydroxy-2,5-naphthalenedisulfonate (Comparative Example 2). The results obtained are shown in Table 7.

**Table 7**

| (Molar absorption coefficient comparison test) | |
|---|---|
| Example or Comparative Example Molar Absorption Coefficient (ε) | |
| Example 6 | 109,000 |
| Example 7 | 96,500 |
| Comparative Example 1 | 82,000 |
| Comparative Example 2 | 24,500 |

### Examples 8, 9, and 10 (near-infrared absorbing filters and heat-resistant stability tests)

In 18.8 parts of MEK was dissolved 1.2 parts of each of the compounds obtained in Examples above. In the dissolved solution was mixed 80 parts of the resin liquid obtained by adding 25 parts of an acrylic resin (Dianal BR-80, from Mitsubishi Rayon Co., Ltd.) into 75 parts of MEK for dissolution to provide a solution for coating. This solution was coated with a thickness of 2 to 4 µm on a polyester film, followed by drying at 80°C to provide the near-infrared absorbing filter of the invention.

The resultant near-infrared absorbing filter was subjected to a heat-resistant stability test in a hot-air drying machine at 80°C for a predetermined amount of time, and also to a moist heat-resistant stability test under conditions of 60°C and 95% RH for a predetermined amount of time. After testing, the filter was subjected to color measuring using a spectrophotometer to calculate L*, a*, and b* values to perform stability evaluation from a change in the b* value. The compound of No. 37 was used in Example 8; No. 49 in Example 9; and No. 73 in Example 10. The results of the heat resistance tests are shown in Table 8.

### Comparative Examples 3 and 4

Filters were prepared and evaluated in the same way as in Examples 8 and 9 except for the use of
N,N,N',N'-tetrakis{p-di(n-butyl)aminophenyl}phenylenediim monium hexafluorophosphate (Comparative Example 3) and
N,N,N',N'-tetrakis{p-di(n-butyl)aminophenyl}phenylenediim monium borate (Comparative Example 4) described in Japanese Patent Publication (KOKOKU) No. 7-51555 (page 2) in place of the above described compounds. The results obtained are shown in Table 8.

**Table 8**

| (Heat-resistant stability test) | | | |
|---|---|---|---|
| b* value | | | |
| Example or Comparative Example | Initial | 4 days after | 14 days after |
| Example 8 | 2.7 | 4.7 | 5.5 |
| Example 9 | 4.2 | 5.8 | 6.5 |
| Example 10 | 4.0 | 5.1 | 5.3 |
| Comparative Example 3 | 2.9 | 6.4 | 9.0 |
| Comparative Example 4 | 3.5 | 11.3 | 14.3 |

### Example 9 (near-infrared absorbing filter)

The compound of No. 37 obtained in Example 1 above was added in an amount of 0.03% to PMMA (polymethyl methacrylate), followed by injection molding at 200°C to provide near-infrared absorbing filters of the invention having thicknesses of 1 mm and 3 mm. The average light transmittances of the resultant filters at 800 to 1,000 nm were determined using a spectrophotometer, demonstrating that they were 20% and 3% in filters with thicknesses of 1 mm and 3 mm, respectively.

Table 7 demonstrates that the near-infrared absorbing compounds used in the invention have molar absorption coefficients of as high as 90,000 or more. Also, Table 8 demonstrates that the near-infrared absorbing filters of the invention containing these compounds are highly excellent in stability under conditions of high temperature and high humidity because they show smaller changes in the b* value relative to those in the comparative samples.

The near-infrared absorbing compounds of the invention are excellent compounds containingno antimony andhavingmolar absorption coefficients of as high as 90,000 or more. In addition, they are excellent in environmental stability, particularly heat resistance compared to conventional diimmonium salts having a hexafluorophosphate ion, a perchlorate ion, or a fluoroborate ion without containing antimony or the like. The near-infrared absorbing filters using these compounds are near-infrared absorbing filters containing no antimony or the like and highly excellent in heat resistance, and produce little coloration in the visible region because it less easily causes reactions such as degradation due to heat. Due to these features, the near-infrared absorbing compounds of the invention can be suitably used in near-infrared absorbing filters or near-infrared absorbing films such as, for example, a heat-insulating film and sunglasses, and particularly fits for a near-infrared absorbing filter for a plasma display.

## Claims

1. A near-infrared absorbing filter **characterized by** comprising a compound consisting of a salt of a cation obtained by oxidation of a substance of formula (1) below and an anion: 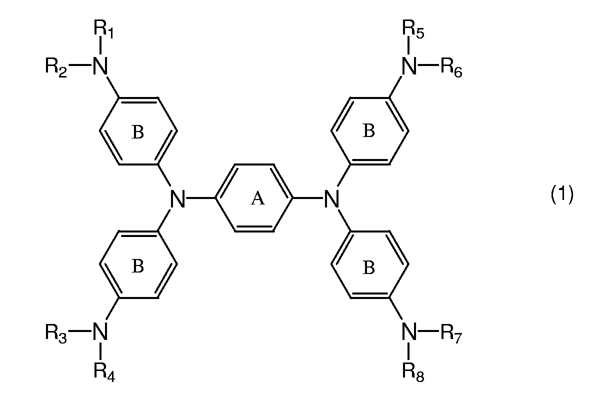 wherein rings A and B may have a substituent (s), and R₁ to R₈ independently represent a substituted or unsubstituted (C1 to C8) alkyl group, cycloalkyl group, alkenyl group or aryl group;
said anion (X) being an alkylsulfonate ion having 1 to 8 carbon atoms, necessary for neutralization of said cation, which may be unsubstituted or substituted with a halogen atom, a lower alkoxy group, cyano group or hydroxyl group.

2. The near-infrared absorbing filter according to claim 1, wherein the compound consisting of a salt of a cation obtained by oxidation of a substance of formula (1) and an anion has a structure of formula (4) below:

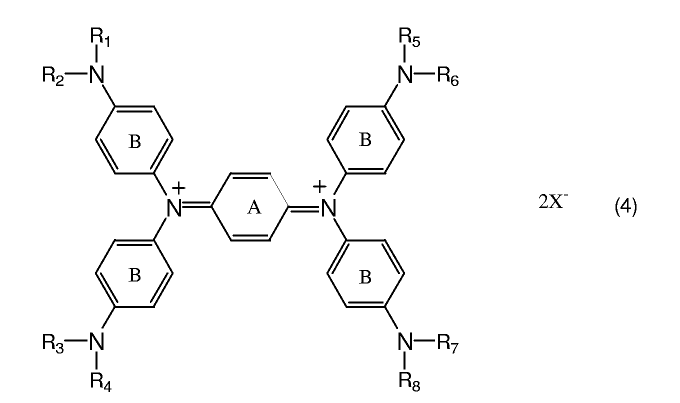

3. The near-infrared absorbing filter according to claim 1 or 2, wherein rings A and B are unsubstituted except in the 1- and 4-positions, or each have 1 to 4 halogen atoms, lower alkyl groups, lower alkoxy groups, cyano groups or hydroxyl groups as substituents.

4. The near-infrared absorbing filter according to any one of claims 1 to 3, wherein X is an alkylsulfonic acid having 1 to 8 carbon atoms which is unsubstituted or substituted with a fluorine atom(s).

5. The near-infrared absorbing filter according to any one of claims 1 to 4, wherein the filter is for use in a plasma display panel.

6. A near-infrared absorbing composition **characterized by** comprising, in a resin, a compound consisting of a salt of a cation obtained by oxidation of a substance of formula (1) and an anion, said anion being an alkylsulfonate ion having 1 to 8 carbon atoms, necessary for neutralization of the cation, which may be unsubstituted or substituted with a halogen atom, a lower alkoxy group, cyano group or hydroxy group.

7. A near-infrared absorbing compound consisting of a salt of a cation obtained by oxidation of a substance of formula (1) below and an anion: 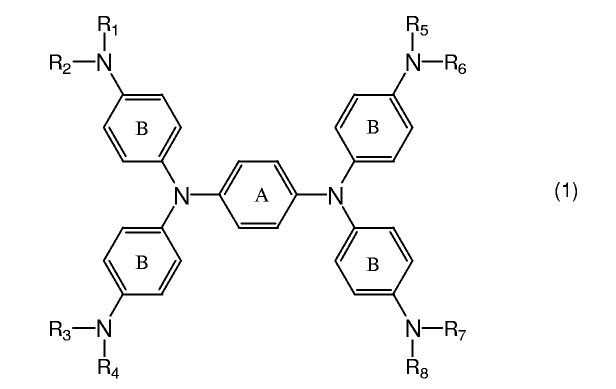 wherein rings A and B may have a substituent (s), and R₁ to R₈ independently represent a substituted or unsubstituted (C1 to C8) alkyl group, cycloalkyl group, alkenyl group or aryl group;
said anion being an alkylsulfonic acid, necessary for neutralization of the cation, represented by formula (2) below: 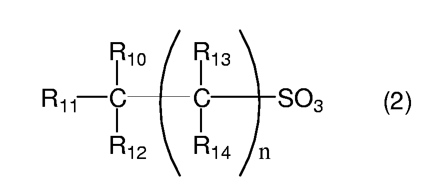 wherein R₁₀ to R₁₄ independently represent a hydrogen or halogen atom, a lower alkyl group, lower alkoxy group, cyano group or hydroxyl group, and n represents an integer of 1 to 7.

8. A near-infrared absorbing compound represented by formula (6) below: 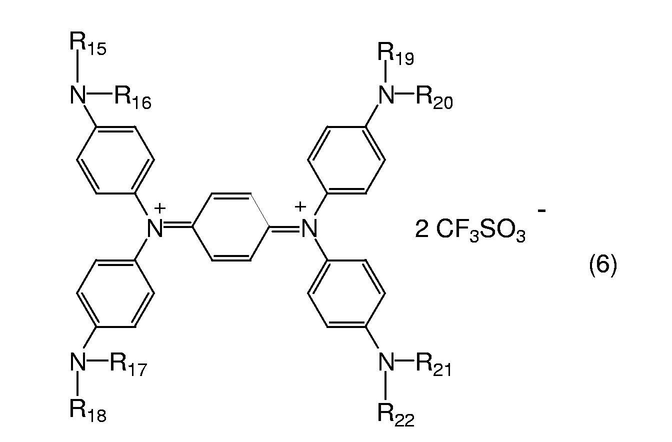 wherein R₁₅ to R₂₂ independently represent a straight-chain or branched butyl or pentyl group.
